(19) Europäisches Patentamt
European Patent Office
Office européen des brevets

(11) Publication number: 0 066 989
B1

(12) **EUROPEAN PATENT SPECIFICATION**

(45) Date of publication of patent specification: 20.03.85

(51) Int. Cl.$^4$: C 07 C 125/065, A 01 N 47/24

(21) Application number: 82302573.9

(22) Date of filing: 20.05.82

(54) **Novel substituted nitrodiphenyl ethers, herbicidal compositions containing them, a process for the preparation of the nitrodiphenyl ethers and the use thereof for combating weeds.**

(30) Priority: 01.06.81 US 269224

(43) Date of publication of application: 15.12.82 Bulletin 82/50

(45) Publication of the grant of the patent: 20.03.85 Bulletin 85/12

(84) Designated Contracting States: AT BE CH DE FR GB IT LI NL SE

(56) References cited: EP-A-0 013 660

(73) Proprietor: Rohm and Haas Company
Independence Mall West
Philadelphia, Pennsylvania 19105 (US)

(72) Inventor: Ashmore, John William
86, Wexford Road
North Wales Pennsylvania 18454 (US)
Inventor: Fujimoto, Ted Tsutomu
44 E. Norton Drive
Churchville Pennsylvania 18966 (US)

(74) Representative: Wood, Peter Worsley Spencer et al
Rohm and Haas Company Patent Department
Chesterfield House Bloomsbury Way
London WC1A 2TP (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 066 989 B1

**Description**

This invention concerns the provision of new nitrodiphenyl ethers, the preparation thereof, herbicidal compositions containing them and methods of combating weeds.

Diphenyl ethers are known to be effective weed control agents. See, for example U.S. Patent Nos. 3,928,416; 3,454,392; 3,798,276; 3,873,303; 4,001,005; and 4,029,493 among the many patents issued. See also U.S. Patent No. 4,200,587 and E. P. 13—360. However, even now herbicidal effectiveness of a diphenyl ether cannot be predicted knowing only the structure and often quite closely related compounds will have quite different weed control abilities. See, *Advances in Agronomy,* Vol. 24, pages 331, 332, 355, 356, 357 and 358, *Herbicides, Chemical Degradation and Mode of Action,* Kearney and Kaufman, Vol. 2, Dekker, Inc. pages 552—563 and 728—737 and *Mode of Action of Herbicides,* Ashton and Crafts and, also, U.S. Patents Nos. 3,454,392 and 3,776,961.

An ideal herbicide should give selective weed control over the full growing season, with a single administration at low rates of application. It should be able to control all common weeds by killing them as the seed, the germinating seed, the seedling, and the growing plant. At the same time, the herbicide should be substantially non-phytotoxic to the crops to which it is applied and should decompose or otherwise be dissipated so as not to poison the soil permanently. The known diphenyl ether herbicides fall short of these ideals and thus the search continues to discover new herbicides which are more selective or which complement the known diphenyl ethers.

In accordance with the present invention, there is provided a class of diphenyl ethers having the formula:

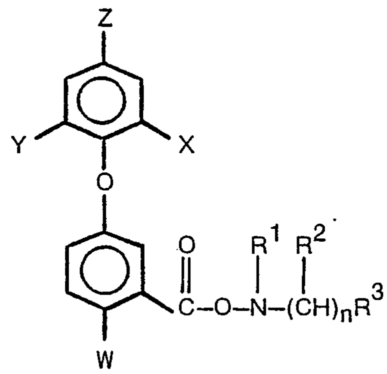

wherein X is hydrogen, halo (viz chloro, fluoro, bromo and iodo), trihalomethyl (e.g. trifluoromethyl), alkyl (e.g. ($C_1$—$C_6$)alkyl), nitro or cyano; Y is hydrogen or halo; Z is halo, trihalomethyl (e.g. trifluoromethyl), pentahaloethyl (e.g. perfluoroethyl); W is nitro, cyano, halo, or a radical of the formula $S(O)_{n'}$ R, wherein R is ($C_1$—$C_6$)alkyl (i.e. $C_1$, $C_2$, $C_3$, $C_4$, $C_5$ and $C_6$ and all isomers of $C_3$—$C_6$) or trihaloalkyl (e.g. trifluoromethyl) n' is 0, 1 or 2; $R^1$ is hydrogen or an optionally substituted saturated or unsaturated hydrocarbyl radical, e.g. alkyl (particularly ($C_1$—$C_6$) alkyl), alkenyl (particularly ($C_3$—$C_5$)alkenyl), or alkynyl (particularly ($C_3$—$C_5$)alkynyl; $R^2$ is hydrogen or ($C_1$—$C_6$)alkyl; n is 0, 1, 2 or 3; $R^3$ is an optionally substituted group which is alkoxycarbonyl (particularly alkoxycarbonyl, wherein the alkoxy moiety has from 1 to 6 carbon atoms), alkylcarbonyl (particularly alkylcarbonyl wherein the alkyl moiety has from 1 to 6 carbon atoms), formyl, aminocarbonyl, mono-nuclear aryloxy (particularly phenoxy) or carboxy (including the agronomically acceptable amides, esters and salts thereof); or $R^1$ and $R^2$ may be joined to form, together with the nitrogen to which they are attached, a heterocyclic ring containing one nitrogen atom and from 4 to 8 nuclear carbon atoms, such as pyrrolidinyl, piperidyl, hexamethyleneiminyl, heptamethyleneiminyl or octamethyleneiminyl.

The term "alkyl" or "alkoxy" includes both the straight and branch chained hydrocarbon groups, such as methyl, ethyl, propyl, isopropyl, butyl, isobutyl, *tert*-butyl, methoxy, ethoxy, propoxy, isopropoxy, butoxy, isobutoxy and *tert*-butoxy.

The terms "alkenyl" and "alkynyl" include both straight and branched chain unsaturated hydrocarbon groups such as allyl, butenyl, isobutenyl, pentenyl, isopentyl, propargyl, butynyl, isobutynyl and pentynyl.

When the $R^1$ and/or $R^3$ radicals are substituted, the substituent(s), which may be the same or different when more than one is present, are those selected from halo, cyano, hydroxy, alkoxy, alkylthio, alkylsulfinyl, alkylsulfonyl, alkylcarbonyl, alkoxycarbonyl, aminocarbonyl, trifluoromethyl, nitro, alkylamino and carboxy (including agronomically acceptable salts, esters and amides thereof).

Preferred compounds of this invention are those wherein $R^1$ is hydrogen, ($C_1$—$C_6$)alkyl, ($C_3$—$C_5$)alkenyl or ($C_3$—$C_5$)alkynyl; $R^2$ is hydrogen; $R^3$ is alkoxycarbonyl or alkylcarbonyl each containing 1 to 6 carbon atoms in the alkoxy or alkyl moiety as the case may be and n is 0 or 1.

A subclass of preferred compounds are those wherein X is chlorine, Y is hydrogen, Z is $CF_3$, W is $NO_2$, $R^1$ is hydrogen or ($C_1$—$C_6$)alkyl, $R^2$ is hydrogen, n is 0 or 1, and $R^3$ is alkoxycarbonyl containing 1 to 6 carbon atoms in the alkoxy moiety.

Preferred compounds are:
(2-methoxycarbonylpyrrolidinyl)-5-(2-methyl-4-trifluoromethylphenoxy)-2-trifluoromethanesulfonylbenzoate;
N-(methoxycarbonylmethyl)amino-5-(2-chloro-4-trifluoromethylphenoxy)-2-nitrobenzoate;
N-methyl-N-(2-ethoxycarbonylethyl)amino-5-(2-nitro-4-pentafluoroethylphenoxy)-2-cyanobenzoate.
N-(carboxymethyl)amino-5-(2-cyano-4-chlorophenoxy)-2-bromobenzoate sodium salt;
N-methoxycarbonylmethyl-N-(2-methoxyethyl)amino-5-(4-trifluoromethylphenoxy)-2-nitrobenzoate;
N-(2-chloroethyl)-N-(2-ethoxycarbonylethyl)amino-5-(2,6-dichloro-4-pentafluoroethylphenoxy)-2-cyanobenzoate;
N-methoxycarbonyl-N-(2-methoxyethyl)amino-5-(2,4-dichlorophenoxy)-2-iodobenzoate;
N-ethoxycarbonyl-N-(2-ethylthioethyl)amino-5-(2-trifluoromethyl-4-bromophenoxy)-2-methanesulfonylbenzoate;
N-ethoxycarbonyl-N-(cyanomethyl)aminooxy-5-(2-methyl-4-trifluoromethylphenoxy)-2-trifluoromethanesulfonylbenzoate;
N-methyl-N-(2-methanesulfonylethyl)amino-5-(2-nitro-4-pentafluoroethylphenoxy)-2-cyanobenzoate;
N-acetylamino-5-(2-chloro-4-trifluoromethylphenoxy)-2-nitrobenzoate;
N-formylamino-5-(2-cyano-4-chlorophenoxy)-2-chlorobenzoate;
N-dimethylaminocarbonyl-N-methylamino-5-(4-trifluoromethylphenoxy)-2-nitrobenzoate;
N-methanesulfonylamino-5-(2-chloro-4-trifluoromethylphenoxy)-2-nitrobenzoate;
N-methanesulfinylamino-5-(2,6-dichloro-4-trifluoromethylphenoxy)-2-cyanobenzoate;
N-(4-chlorophenyl)amino-5-(2,4-dichlorophenoxy)-2-nitrobenzoate;
N-methoxycarbonylamino-5-(2-chloro-4-trifluoromethylphenoxy)-2-nitrobenzoate;
N-ethoxycarbonyl-N-methylamino-5-(2-chloro-4-trifluoromethylphenoxy)-2-nitrobenzoate;
N-ethoxycarbonyl-N-allylamino-5-(2-chloro-4-trifluoromethylphenoxy)-2-nitrobenzoate;
N-methoxycarbonyl-N-(3-ethoxycarbonyl-2-propenyl)amino-5-(4-trifluoromethylphenoxy)-2-nitrobenzoate;
N-propoxycarbonyl-N-(2-propynyl)amino-5-(2,6-dichloro-4-pentafluoroethylphenoxy)-2-cyanobenzoate;
N-(2-propoxycarbonyl)-N-(2-chloroethyl)amino-5-(2,4-dichlorophenoxy)-2-iodobenzoate;
N-(*tert*-butoxycarbonyl)-N-(methoxycarbonylmethyl)amino-5-(2-trifluoromethyl-4-chlorophenoxy)-2-methanesulfonylbenzoate;
N-(ethoxycarbonylmethyl)amino-5-(2-fluoro-4-chlorophenoxy)-2-methanesulfonylbenzoate;
N-(1-propoxycarbonylethyl)-N-(2-propenyl)-amino-5-(2-methyl-4-chlorophenoxy)-2-nitrobenzoate;
N-(3-t-butoxycarbonylpropyl)-N-(2-propynyl)amino-5-(2-nitro-4-chlorophenoxy)-2-iodobenzoate;
N-methoxycarbonyl-N-methylamino-5-(2-cyano-4-trifluoromethylphenoxy)-2-nitrobenzoate;
N-methoxycarbonyl-N-methylamino-5-(2,6-dichloro-4-trifluoromethylphenoxy)-2-cyanobenzoate; and
N-acetyl-N-methylamino-5-(2,4,6-trichlorophenoxy)-2-nitrobenzoate.

In general, the diphenyl ethers of this invention, (I, supra) may be prepared by reacting an appropriately substituted diphenyl ether (II, infra) with an appropriate hydroxyl amine (III, infra) as illustrated by the following reaction:

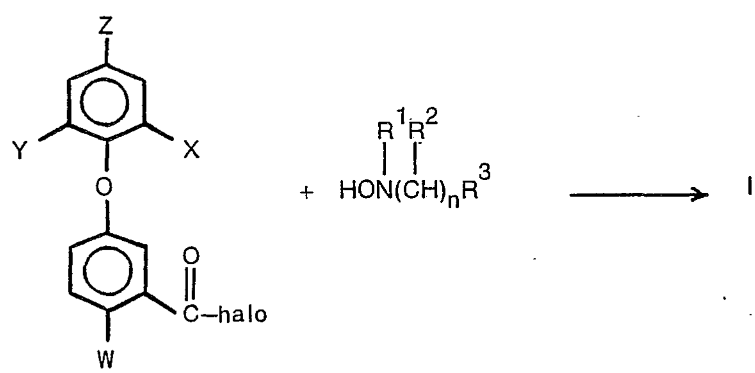

II III

wherein W, X, Y, Z, $R^1$, $R^2$, $R^3$ and n are as defined above and halo is preferably chloro.

This reaction is conducted at a temperature in the range of from about 0° to 50°C in a suitably inert solvent for example, an ether, ketone or a halogenated hydrocarbon. Typical solvent are, tetrahydrofuran, diethyl ether, dichloromethane, chloroform, carbon tetrachloride, acetone and methylethyl ketone. A base is employed as an acid scavenger such as aromatic or tertiary amines including pyridine, lutidene, collidene, triethyl- or trimethyl-amine.

The hydroxyl amines (III, supra) are either known compounds or can be prepared by the following procedure in *Houben-Weyl Methoden der Organishen Chemie 10/1* p. 1097.

The diphenyl ethers (I, supra) of the invention are useful as pre-emergence and postemergence herbicides. Pre-emergence herbicides are ordinarily used to treat the soil by application either before seeding, during seeding, or after seeding and before the crop emerges. Post-emergence herbicides are those which are applied after the plants have emerged. The compounds of this invention are especially active as pre-emergence herbicides. Thus the invention provides a method of combating weeds which comprises applying to weeds, particularly weed seedlings, or to the surface of the growth medium prior to emergence of weeds, one or more of the diphenyl ethers in an amount sufficient to combat the growth of the weeds.

Among the crops on which the diphenyl ethers of the inventioncan be advantageously employed are: cotton, soybeans, peanuts, beans, peas, carrots, corn, wheat, rice, and other cereal crops.

The diphenyl ethers (I, *supra*) can be applied in any amount which will give the required control of weeds. A standard rate of application of the herbicides of the invention is in the range of from about 0.022 to about 13.5 kg/ha (0.02 to 12 lb/A). A preferred range is from about 0.1 to about 4.5 kg/ha (0.1 to about 4 lb/A).

Under some conditions, the diphenyl ethers (I, *supra*) may be advantageously incorporated into the soil or other growth medium prior to planting a crop. This incorporation can be by any convenient means, including simple mixing with the soil, applying the diphenyl ether to the surface of the soil and then discing or dragging into the soil to the desired depth, or by employing a liquid carrier.

One or more of the diphenyl ethers of the invention can be applied to the growth medium or to plants to be treated either alone or as a component in a herbicidal composition or formulation which also comprises an agronomically acceptable carrier or diluent. "Agronomically acceptable carrier or diluent" is any carrier or diluent which can be used to dilute, dissolve, disperse or diffuse a herbicidal compound in the composition without impairing the effectiveness of the herbicidal compound and which by itself has no permanent detrimental effect on the soil, equipment, crops, or agronomic environment. Usually the herbicidal composition will contain a surfactant.

The herbicidal compositions can be either solid or liquid formulations or solutions, e.g. solutions in any organic solvent serving as a carrier. For example, the diphenyl ethers can be formulated as wettable powders, emulsifiable concentrates, dusts, granular formulations, aerosols, or flowable emulsion concentrates. In such formulations, the compounds are extended with a liquid or solid carrier and, when desired, suitable surfactants are incorporated.

It is usually desirable, particularly in post-emergence applications, to include adjuvants, such as wetting agents, spreading agents, dispersing agents, stickers, adhesives, and the like, in accordance with usual agricultural practices. Examples of adjuvants which are commonly used in the art can be found in the John W. McCutcheon, Inc. publication "Detergents and Emulsifiers 1969 Annual".

Formulations of the diphenyl ethers may be prepared as described in Applicant's European Patent Application, Publication No. 0 020 052 which corresponds to Australian Application No. 58498/80. The diphenyl ethers may be used in conjunction with other herbicides as listed in Publication No. 0 020 052 and may be applied as there described.

The following examples will further illustrate this invention.

### Example 1

N-Ethoxycarbonylamino-5-(2-chloro-4-trifluoromethylphenoxy)-2-nitrobenzoate

To an ice-cooled solution of 5-(2-chloro-4-trifluoromethylphenoxy)-2-nitrobenzoyl chloride (7.6 g., 20 mmol.) in 70 ml. dichlorimethane was added pyridine (2.5 g., 30 mmol). A mild exothermic reaction ensued and a voluminous precipitate formed. The slurry was stirred an additional 30 minutes with ice bath cooling then N-hydroxyurethane (2.3 g., 22 mmol.) was added. The reaction mixture became homogeneous, the ice bath was removed and the reaction mixture stirred overnight. The reaction mixture was worked-up by washing first with 3M HCL (2 × 30 ml.), then with a saturated aqueous solution of $NaHCO_3$ (30 ml.) and drying the organic phase with anhydrous $MgSO_4$. Concentration under reduced pressure afforded 9.0 g. of an off-white solid. Crystallization of the crude product with ethyl acetate/hexane afforded 4.7 g. of an off-white solid, m.p. 125°—128°C.

### Example 2

N-Ethoxycarbonyl-N-methylamino-5-(2-chloro-4-trifluoromethylphenoxy)-2-nitrobenzoate

By following substantially the procedure of Example 1 and by employing the following amounts of:

7.6 g, (20 mmol.) of 5-(2-chloro-4-trifluoromethylphenoxy)-2-nitrobenzoylchloride;
2.5 g, (30 mmol.) of pyridine;
2.5 g, (21 mmol.) of N-hydroxy-N-methylurethane; and
20 ml. of dichloromethane

there was obtained 6.3 g of product as off-white solid m.p. 80°—83°C.

*Herbicidal activity*

The compounds of Examples 1 and 2 were evaluated on the following representative species:

| | | | Approx. No. Seeds |
|---|---|---|---|
| Monocots: | Barnyardgrass | (Echinochloa crusgalli) | 25 |
| | Downybrome | (Bromus tectorum) | 20 |
| | Foxtail | (Setaria spp) | 25 |
| | Johnsongrass | (Sorghum Halepense) | 25 |
| | Nutsedge | (Cyperus esculentus) | 5 |
| | Wild Oat | (Avena fatura) | 20 |
| Dicots: | Cocklebur | (Xanthium pensylvanicum) | 3 |
| | Marigold | (Tagetes spp) | 15 |
| | Morning Glory | (Ipomoea spp) | 10 |
| | Tomato | (Lycopersicon esculentum) | 15 |
| | Velvetleaf | (Abutilon theophrasti) | 15 |
| | Sickle pod | (Cassia obtusifolia) | 6 |

The following test procedure was employed. Seeds of the above species were planted in soil in trays (approximately 18 cm × 27 cm × 8 cm. For pre-emergence tests, the trays were sprayed with the test compound immediately after planting. For post-emergence tests, the seeds were allowed to germinate and after growing in the greenhouse for two weeks, the growing plants were treated with the test compound. The compound to be evaluated was dissolved in acetone and/or water and sprayed over the trays using a carrier volume equivalent to 468 l/ha (50 U.S. gallons per acre) at the dose (in pounds per acre, lb/A) specified in the table. About two weeks after application of the test compound, the state of growth of the plants is observed and the phytotoxic effect of each compound determined as follows: each species is evaluated on a scale of 0—100 in which 0 = no activity and 100 = total kill and the results for the monocots (M) and dicots (D) separately averaged. The following table shows the results obtained for the compounds of the invention at 2 lb/A., 1/2 lb/A. and 1/8 lb./A which correspond to 2.24 kg/ha, 0.56 kg/ha and 0.14 kg/ha.

HERBICIDAL DATA

| | PRE | | | | | |
|---|---|---|---|---|---|---|
| | 1/8 | | 1/2 | | 2 | |
| | M | D | M | D | M | D |
| Ex. 1 | 48 | 65 | 74 | 83 | 83 | 98 |
| Ex. 2 | 6 | 20 | 26 | 51 | 48 | 62 |

| | POST | | | | | |
|---|---|---|---|---|---|---|
| | 1/8 | | 1/2 | | 2 | |
| | M | D | M | D | M | D |
| Ex. 1 | 13 | 34 | 20 | 64 | 48 | 79 |
| Ex. 2 | 2 | 23 | 13 | 50 | 24 | 71 |

In conclusion there may be singled out for mentioned the following compounds of Formula I, viz. all the individual compounds of Formula I hereinbefore disclosed together with the additional compounds specified in paragraphs (b) to (i) below but in which (referring to the symbols used in Formula I):

(a) X in the individual ether in question is replaced by a different group or atom selected from each and every one of H, Br, Cl, F, I, $CF_3$, $CCl_3$, $CH_3$, $C_2H_5$, $C_3H_7$, $C_4H_9$, $C_5H_{11}$, $C_6H_{13}$ (including each and every isomer of the last 4 groups), $NO_2$ and CN;

(b) Y in the individual ether in question is replaced by a different atom selected from each and every one of H, Br, Cl, F and I;

(c) Z in the individual ether in question is replaced by a different group or atom selected from each and every one of Br, Cl, F, I, $CF_3$, $CCl_3$, $C_2F_5$ and $C_2Cl_5$;

(d) W in the individual ether in question is replaced by a different group selected from each and every one of $NO_2$, CN, Br, Cl, F, I, $CF_3$, $CCl_3$ and $-S(O)_{n'}R$ where n' is each and every one of 0, 1 and 2 and R, for each value of n', is each and every one of the alkyl groups listed in (a) above;

(e) $R^1$ in the individual ether in question is replaced by a different group or atom selected from each and every one of H, each and every one of the alkyl groups listed in (a) above and $C_3$-, $C_4$- and $C_5$-alkenyl or -alkynyl including each and every isomer thereof;

(f) the group

$$\overset{R^2}{|}$$
$$-(CH)_2-$$

in the individual ether in question is replaced by each and every group, wherein n is each and every one of 0, 1, 2 and 3 and $R^2$, for each value of n, is H or each and every one of the alkyl groups listed in (a) above;

(g) the group

$$\overset{R^1}{|}\quad\overset{R^2}{|}$$
$$-N-(CH)_n-$$

in the individual ether in question is replaced by each and every residue of a group of the formula

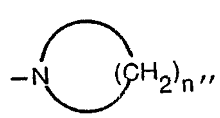

where n'' is 4, 5, 6, 7 or 8;

(h) $R^3$ in the individual ether in question is replaced by each and every one of the groups selected from (a) alkoxycarbonyl and alkylcarbonyl in each of which the alkyl moiety is each and every one of the alkyl groups listed in a) above, b) formyl, c) aminocarbonyl, d) phenoxy and e) carboxy (including the agronomically acceptable amides, esters (notably alkyl esters, "alkyl" being each and every one of the alkyl groups listed in (a) above) and salts (notably the K, Na, Mg and Ca salts) thereof; and

(i) $R^1$ and/or $R^3$ in each of the groups defined in (e), (g) or (h) above is a group which is substituted by one or more of the same or different substituents selected from Cl, F, Br, I, CN, OH, $(C_1—C_6)$alkoxy, $(C_1—C_6)$alkylthio, $(C_1—C_6)$alkylsulfinyl, $(C_1—C_6)$alkylsulfonyl, alkyl$(C_1—C_6)$carbonyl, alkoxy$(C_1—C_6)$carbonyl, $CF_3$, nitro, $(C_1—C_6)$alkylamino and carboxy (including the carboxy derivatives listed in paragraph (h), (e) above.

**Claims for the Contracting States: BE CH DE FR GB IT LI NL SE**

1. A compound of the formula

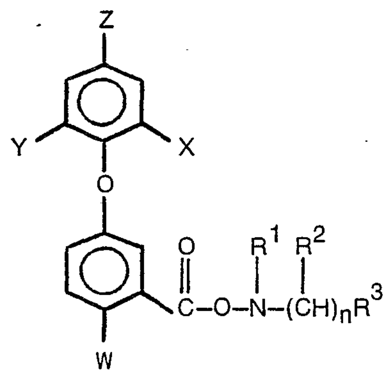

wherein X is hydrogen, halo, trihalomethyl, alkyl, nitro or cyano; Y is hydrogen or halo; Z is halo, trihalomethyl or pentahaloethyl; W is nitro, cyano, halo or a radical of the formula $S(O)_{n'}R$ wherein R is $(C_1—C_6)$alkyl or trihaloalkyl, n' is 0, 1 or 2; $R^1$ is hydrogen or an optionally substituted saturated or unsaturated hydrocarbyl radical; $R^2$ is hydrogen or $(C_1—C_6)$alkyl; n is 0, 1, 2 or 3; $R^3$ is an optionally substituted group which is alkoxycarbonyl, alkylcarbonyl, formyl aminocarbonyl, mononuclear aryloxy or carboxy (including agronomically acceptable amides, esters or salts thereof); or $R^1$ and $R^2$ may be joined to form, together with the nitrogen to which they are attached, a heterocyclic ring containing one nitrogen atom and from 4 to 8 nuclear carbon atoms.

2. A compound as claimed in Claim 1, wherein $R^1$ is hydrogen, $(C_1—C_6)$alkyl, $(C_3—C_5)$alkenyl or $(C_3—C_5)$alkynyl; $R^2$ is hydrogen and $R^3$ is alkoxycarbonyl or alkylcarbonyl each containing 1 to 6 carbon atoms in the alkoxy or alkyl moiety as the case may be and n is 0 or 1.

3. A compound as claimed in Claim 1, wherein X is chlorine, Y is hydrogen, Z is $CF_3$, W is $NO_2$, $R^1$ is hydrogen or $(C_1—C_6)$alkyl, $R^2$ is hydrogen, n is 0 or 1 and $R^3$ is alkoxycarbonyl containing 1 to 6 carbon atoms in the alkoxy moiety.

4. A compound as claimed in Claim 3, wherein $R^1$ is hydrogen or methyl, n is 0 and $R^3$ is ethoxycarbonyl.

5. A method of combating weeds which comprises applying to weed seedlings, or the surface of the growth medium prior to the emergence of weeds therefrom, a compound as claimed in any one of Claims 1—4 in an amount sufficient to combat the growth of the weeds.

6. A herbicidal composition containing, as active ingredient, at least one compound as claimed in any one of Claims 1—4 and an agronomically acceptable carrier or diluent for the active ingredient, the composition optionally also containing a surfactant.

7. A process for preparing a compound as claimed in Claim 1 which comprises reacting a compound of the formula:

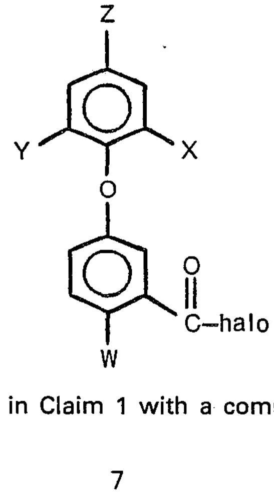

wherein W, X, Y and Z are as defined in Claim 1 with a compound of the formula: $HONR^1(CHR^2)_nR^3$;

wherein $R^1$, $R^2$, $R^3$ and n are as defined in Claim 1, at a temperature in the range of from about 0°C. to about 50°C. in an inert solvent and in the presence of an acid scavenger.

**Claims for the Contracting State: AT**

1. A herbicidal composition containing an active herbicidal component and an agronomically acceptable diluent or carrier therefor, wherein the active component comprises one or more diphenyl ethers of the formula:

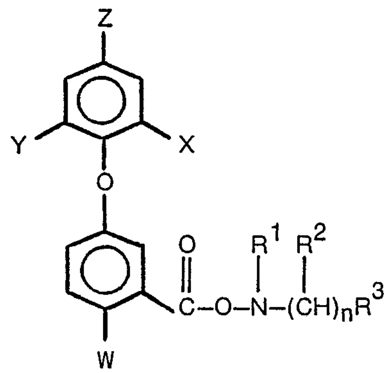

wherein X is hydrogen, halo, trihalomethyl, alkyl, nitro or cyano; Y is hydrogen or halo; Z is halo, trihalomethyl or pentahaloethyl; W is nitro, cyano, halo or a radical of the formula $S(O)_{n'}R$ wherein R is ($C_1$—$C_6$)alkyl or trihaloalkyl, n' is 0, 1 or 2; $R^1$ is hydrogen or an optionally substituted saturated or unsaturated hydrocarbyl radical; $R^2$ is hydrogen or ($C_1$—$C_6$)alkyl; n is 0, 1, 2 or 3; $R^3$ is an optionally substituted group which is alkoxycarbonyl, alkylcarbonyl, formyl aminocarbonyl, mononuclear aryloxy or carboxy (including agronomically acceptable amides, esters or salts thereof); or $R^1$ and $R^2$ may be joined to form, together with the nitrogen to which they are attached, a heterocyclic ring containing one nitrogen atom and from 4 to 8 nuclear carbon atoms.

2. A composition as claimed in Claim 1, wherein $R^1$ is hydrogen, ($C_1$—$C_6$)alkyl, ($C_3$—$C_5$)alkenyl or ($C_3$—$C_5$)alkynyl; $R^2$ is hydrogen and $R^3$ is alkoxycarbonyl or alkylcarbonyl each containing 1 to 6 carbon atoms in the alkoxy or alkyl moiety as the case may be and n is 0 or 1.

3. A composition as claimed in Claim 1, wherein X is chlorine, Y is hydrogen, Z is $CF_3$, W is $NO_2$, $R^1$ is hydrogen or ($C_1$—$C_6$)alkyl, $R^2$ is hydrogen, n is 0 or 1 and $R^3$ is alkoxycarbonyl containing 1 to 6 carbon atoms in the alkoxy moiety.

4. A composition as claimed in Claim 3, wherein $R^1$ is hydrogen or methyl, n is 0 and $R^3$ is ethoxycarbonyl.

5. A method of combating weeds which comprises applying to weed seedlings, or the surface of the growth medium prior to the emergence of weeds therefrom, a composition as claimed in any one of Claims 1—4 in an amount sufficient to combat the growth of the weeds.

6. A process for preparing a diphenyl ether as defined in Claim 1 which comprises reacting a compound of the formula:

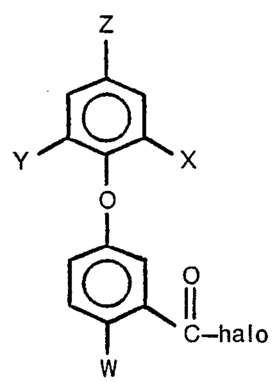

wherein W, X, Y and Z are as defined in Claim 1 with a compound of the formula: $HONR^1(CHR^2)_nR^3$; wherein $R^1$, $R^2$, $R^3$ and n are as defined in Claim 1, at a temperature in the range of from about 0°C. to about 50°C. in an inert solvent and in the presence of an acid scavenger.

**Patentansprüche für die Vertragsstaaten: BE CH DE FR GB IT LI NL SE**

1. Verbindung der Formel

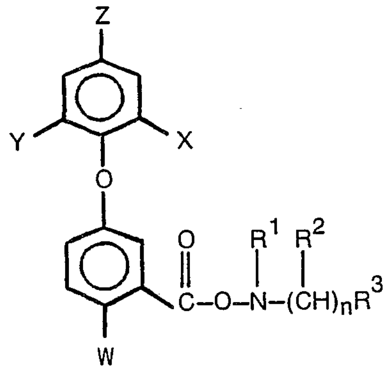

worin X für Wasserstoff, Halogen, Trihalogenmethyl, Alkyl, Nitro oder Cyano steht, Y Wasserstoff oder Halogen ist, Z Halogen, Trihalogenmethyl oder Pentahalogenethyl bedeutet, W Nitro, Cyano, Halogen oder ein Rest der Formel $S(O)_{n'}R$ ist, wobei R für $(C_1—C_6)$Alkyl oder Trihalogenalkyl steht, n' 0, 1 oder 2 ist, $R^1$ Wasserstoff oder ein gegebenenfalls substituierter gesättigter oder nichtgesättigter Kohlenwasserstoffrest ist, $R^2$ Wasserstoff oder $(C_1—C_6)$Alkyl bedeutet, n 0, 1, 2 oder 3 ist, $R^3$ eine gegebenenfalls substituierte Gruppe ist, bei der es sich um Alkoxycarbonyl, Alkylcarbonyl, Formylaminocarbonyl, einkerniges Aryloxy oder Carboxy (einschließlich landwirtschaftlich verträglicher Amide, Ester oder Salze davon) handelt, oder $R^1$ und $R^2$ zusammen verbunden sein können und zusammen mit dem Stickstoffatom, mit dem sie verknüpft sind, einen heterocyclischen Ring bilden, der ein Stickstoffatom und 4 bis 8 Kernkohlenstoffatome enthält.

2. Verbindung nach Anspruch 1, dadurch gekennzeichnet, daß $R^1$ für Wasserstoff, $(C_1—C_6)$Alkyl, $(C_3—C_5)$Alkenyl und $(C_3—C_5)$Alkinyl steht, $R^2$ Wasserstoff ist und $R^3$ Alkoxycarbonyl oder Alkylcarbonyl, jeweils enthaltend 1 bis 6 Kohlenstoffatome in dem Alkoxy- oder Alkylanteil, ist, und n 0 oder 1 ist.

3. Verbindung nach Anspruch 1, dadurch gekennzeichnet, daß X für Chlor steht, Y Wasserstoff ist, Z $CF_3$ bedeutet, W $NO_2$ ist, $R^1$ Wasserstoff oder $(C_1—C_6)$Alkyl darstellt, $R^2$ Wasserstoff ist, n O oder 1 ist, und $R^3$ Alkoxycarbonyl ist, das 1 bis 6 Kohlenstoffatome in dem Alkoxyanteil enthält.

4. Verbindung nach Anspruch 3, dadurch gekennzeichnet, daß $R^1$ für Wasserstoff oder Methyl steht, n 0 ist und $R^3$ Ethoxycarbonyl bedeutet.

5. Verfahren zum Bekämpfen von Unkräutern, dadurch gekennzeichnet, daß auf Unkrautsämlinge oder auf die Oberfläche des Wachstumsmediums vor dem Auflaufen der Unkräuter eine Verbindung gemäß einem der Ansprüche 1 bis 4 in einer Menge aufgebracht wird, die dazu ausreicht, das Wachstum der Unkräuter zu bekämpfen.

6. Herbizides Mittel, das als Wirkstoff wenigstens eine Verbindung gemäß einem der Ansprüche 1 bis 4 und einen für landwirtschaftliche Zwecke verträglichen Träger oder ein Verdünnungsmittel für den Wirkstoff enthält, wobei das Mittel gegebenenfalls auch ein grenzflächenaktives Mittel enthält.

7. Verfahren zur Herstellung einer Verbindung gemäß Anspruch 1, dadurch gekennzeichnet, daß eine Verbindung der Formel

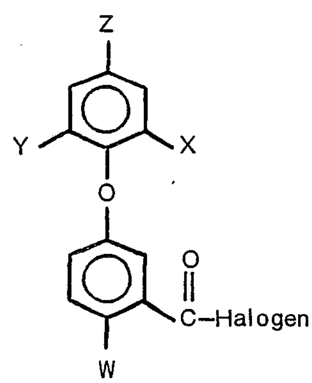

worin W, X, Y und Z die in Anspruch 1 angegebenen Bedeutungen besitzen, mit einer Verbindung der Formel $HONR^1(CHR^2)_nR^3$, worin $R^1$, $R^2$, $R^3$ und n die in Anspruch 1 angegebenen Bedeutungen besitzen, bei einer Temperatur zwischen ungefähr 0°C und ungefähr 50°C in einem inerten Lösungsmittel sowie in Gegenwart eines Säureabfängers umgesetzt werden.

**Patentansprüche für den Vertragsstaat: AT**

1. Herbizides Mittel, das eine aktive herbizide Komponente und ein landwirtschaftlich verträgliches Verdünnungsmittel oder Träger dafür enthält, wobei die aktive Komponente aus einem oder mehreren Diphenylether(n) der Formel

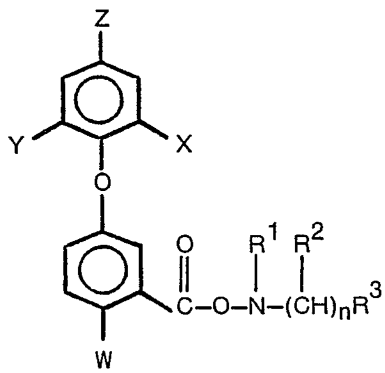

besteht, worin X Wasserstoff, Halogen, Trihalogenmethyl, Alkyl, Nitro oder Cyano bedeutet, Y Wasserstoff oder Halogen ist, Z Halogen, Trihalogenmethyl oder Pentahalogenethyl darstellt, W Nitro, Cyano, Halogen oder einen Rest der Formel $S(O)_{n'}R$ bedeutet, wobei R für $(C_1—C_6)$Alkyl oder Trihalogenalkyl steht, n' 0, 1 oder 2 ist, $R^1$ Wasserstoff oder ein gegebenenfalls substituierter gesättigter oder ungesättigter Kohlenwasserstoffrest ist, $R^2$ Wasserstoff oder $(C_1—C_6)$Alkyl ist, n 0, 1, 2 oder 3 ist, $R^3$ eine gegebenenfalls substituierte Gruppe ist, bei der es sich um Alkoxycarbonyl, Alkylcarbonyl, Formylaminocarbonyl, einkerniges Aryloxy oder Carboxy (einschließlich der für landwirtschaftlich Zwecke verträglichen Amide, Ester oder Salze davon) handelt, oder $R^1$ und $R^2$ miteinander verbunden sein können und zusammen mit dem Stickstoffatom, mit dem sie verknüpft sind, einen heterocyclischen Ring bilden, der ein Stickstoffatom und 4 bis 8 Kernkohlenstoffatome enthält.

2. Mittel nach Anspruch 1, dadurch gekennzeichnet, daß $R^1$ für Wasserstoff, $(C_1—C_6)$Alkyl, $(C_3—C_5)$Alkenyl oder $(C_3—C_5)$Alkinyl steht, $R^2$ Wasserstoff ist und $R^3$ Alkoxycarbonyl oder Alkylcarbonyl ist, jeweils enthaltend 1 bis 6 Kohlenstoffatome in dem Alkoxy- oder Alkylanteil, und n 0 oder 1 bedeutet.

3. Mittel nach Anspruch 1, dadurch gekennzeichnet, daß X für Chlor steht, Y Wasserstoff ist, Z $CF_3$ bedeutet, W $NO_2$ ist, $R^1$ Wasserstoff oder $(C_1—C_6)$Alkyl darstellt, $R^2$ Wasserstoff ist, n O oder 1 ist, und $R^3$ Alkoxycarbonyl ist, das 1 bis 6 Kohlenstoffatome in dem Alkoxyanteil enthält.

4. Mittel nach Anspruch 3, dadurch gekennzeichnet, daß $R^1$ Wasserstoff oder Methyl ist, n 0 ist und $R^3$ Ethoxycarbonyl bedeutet.

5. Verfahren zum Bekämpfen von Unkräutern, dadurch gekennzeichnet, daß auf Unkrautsämlinge oder auf die Oberfläche des Wachstumsmediums vor dem Auflaufen der Unkräuter ein Mittel gemäß einem der Ansprüche 1 bis 4 in einer Menge aufgebracht wird, die dazu ausreicht, das Wachstum der Unkräuter zu bekämpfen.

6. Verfahren zur Herstellung eines Diphenylethers gemäß Anspruch 1, dadurch gekennzeichnet, daß eine Verbindung der Formel

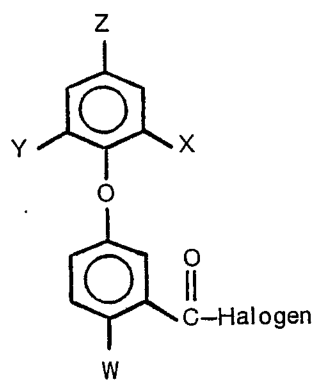

worin W, X, Y und Z die in Anspruch 1 angegebenen Bedeutungen besitzen, mit einer Verbindung der Formel $HONR^1(CHR^2)_nR^3$, worin $R^1$, $R^2$, $R^3$ und n die in Anspruch 1 angegebenen Bedeutungen besitzen, bei einer Temperatur zwischen ungefähr 0°C und ungefähr 50°C in einem inerten Lösungsmittel in Gegenwart eines Säureabfängers umgesetzt wird.

**Revendications pour les Etats Contractants: BE CH DE FR GB IT LI NL SE**

1. Composé de formule

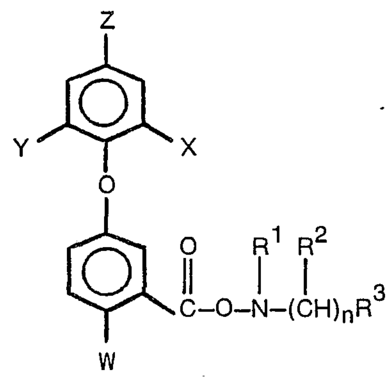

dans laquelle X est un hydrogène, un halogéno, un trihalogéno-méthyle, un alcoyle, un nitro ou un cyano; Y est un hydrogène ou un halogéno; Z est un halogéno, un trihalogénométhyle ou un pentahalogénoéthyle; W est un nitro, un cyano, un halogéno ou un radical de formule $S(O)_{n'}R$, dans laquelle R est un alcoyle en $C_1$—$C_6$ ou un trihalogénoalcoyle, n' est 0, 1 ou 2; $R^1$ est un hydrogène ou un radical hydrocarbyle saturé ou insaturé éventuellement substitué; $R^2$ est un hydrogène ou un alcoyle en $C_1$—$C_6$; n est 0, 1, 2, ou 3; $R^3$ est un groupe éventuellement substitué qui est un alcoxycarbonyle, un alcoylcarbonyle, un formyle, un aminocarbonyle, un aryloxy mononucléaire ou un carboxy (y compris les amides, esters ou sels correspondants acceptables en agronomie); ou $R^1$ et $R^2$ peuvent être unis pour former ensemble avec l'azote auquel ils sont fixés un noyau hétérocyclique contenant un atome d'azote et de 4 à 8 atomes de carbone nucléaires.

2. Composé comme revendiqué dans la revendication 1, dans lequel $R^1$ est un hydrogène, un alcoyle en $C_1$—$C_6$, un alcényle en $C_3$—$C_5$ ou un alcynyle en $C_3$—$C_5$; $R^2$ est un hydrogène et $R^3$ est un alcoxycarbonyle ou un alcoylcarbonyle contenant chacun 1 à 6 atomes de carbone dans le fragment alcoxy ou alcoyle selon le cas, et n est 0 ou 1.

3. Composé comme revendiqué dans la revendication 1, dans lequel X est un chlore, Y est un hydrogène, Z est $CF_3$, W est $NO_2$, $R^1$ est un hydrogène ou un alcoyle en $C_1$—$C_6$, $R^2$ est un hydrogène, n est 0 ou 1, et $R^3$ est un alcoxycarbonyle contenant 1 à 6 atomes de carbone dans le fragment alcoxy.

4. Composé comme revendiqué dans la revendication 3, dans lequel $R^1$ est un hydrogène ou un méthyle, n est 0 et $R^3$ est un éthoxycarbonyle.

5. Procédé de lutte contre les mauvaises herbes qui comprend l'application aux pousses des mauvaises herbes ou à la surface du milieu de culture avant la levée des mauvaises herbes d'un composé comme revendiqué dans l'une quelconque des revendications 1 à 4 en une quantité suffisante pour lutter contre la croissance des mauvaises herbes.

6. Composition herbicide contenant, comme ingrédient actif, au moins un composé comme revendiqué dans l'une quelconque des revendications 1 à 4 et un support ou diluant acceptable en agronomie de l'ingrédient actif, la composition contenant éventuellement également un agent tensio-actif.

7. Procédé pour préparer un composé comme revendiqué dans la revendication 1, qui comprend la réaction d'un composé de formule

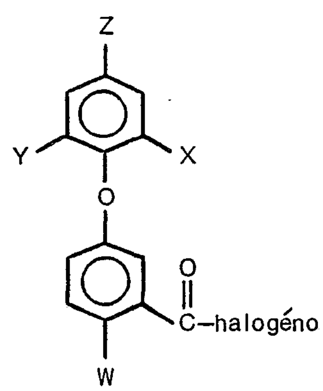

dans laquelle W, X, Y et Z sont comme défini dans la revendication 1, avec un composé de formule: $HONR^1(CHR^2)_nR^3$; dans laquelle $R^1$, $R^2$, $R^3$ et n sont comme défini dans la revendication 1, à une température dans la gamme d'environ 0°C à environ 50°C dans un solvant inerte et en présence d'un fixateur d'acide.

**Revendications pour l'Etat Contractant: AT**

1. Composition herbicide contenant un composant herbicide actif et un support ou diluant acceptable en agronomie de celui-ci, dans laquelle le composant actif comprend un ou plusieurs éthers diphényliques de formule

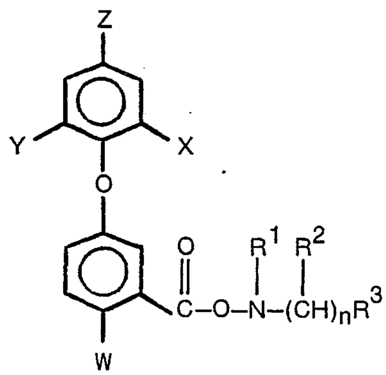

dans laquelle X est un hydrogène, un halogéno, un trihalogéno-méthyle, un alcoyle, un nitro ou un cyano; Y est un hydrogène ou un halogéno; Z est un halogéno, un trihalogénométhyle ou un pentahalogénoéthyle; W est un nitro, un cyano, un halogéno ou un radical de formule $S(O)_{n'}R$, dans laquelle R est un alcoyle en $C_1$—$C_6$ ou un trihalogénoalcoyle, n' est 0, 1 ou 2; $R^1$ est un hydrogène ou un radical hydrocarbyle saturé ou insaturé éventuellement substitué; $R^2$ est un hydrogène ou un alcoyle en $C_1$—$C_6$; n est 0, 1, 2, ou 3; $R^3$ est un groupe éventuellement substitué qui est un alcoxycarbonyle, un alcoylcarbonyle, un formyle, un aminocarbonyle, un aryloxy mononucléaire ou un carboxy (y compris les amides, esters ou sels correspondants acceptables en agronomie); ou $R^1$ et $R^2$ peuvent être unis pour former ensemble avec l'azote auquel ils sont fixés un noyau hétérocyclique contenant un atome d'azote et de 4 à 8 atomes de carbone nucléaires.

2. Composition comme revendiqué dans la revendication 1, dans laquelle $R^1$ est un hydrogène, un alcoyle en $C_1$—$C_6$, un alcényle en $C_3$—$C_5$ ou un alcynyle en $C_3$—$C_5$; $R^2$ est un hydrogéne et $R^3$ est un alcoxycarbonyle ou un alcoylcarbonyle contenant chacun 1 à 6 atomes de carbone dans le fragment alcoxy ou alcoyle selon le cas, et n est 0 ou 1.

3. Composition comme revendiqué dans la revendication 1, dans laquelle X est un chlore, Y est un hydrogène, Z est $CF_3$, W est $NO_2$, $R^1$ est un hydrogène ou un alcoyle en $C_1$—$C_6$, $R^2$ est un hydrogène, n est 0 ou 1, et $R^3$ est un alcoxycarbonyle contenant 1 à 6 atomes de carbone dans le fragment alcoxy.

4. Composition comme revendiqué dans la revendication 3, dans laquelle $R^1$ est un hydrogène ou un méthyle, n est 0 et $R^3$ est un éthoxycarbonyle.

5. Procédé de lutte contre les mauvaises herbes qui comprend l'application aux pousses des mauvaises herbes ou à la surface du milieu de culture avant la levée des mauvaises herbes d'une composition comme revendiqué dans l'une quelconque des revendications 1 à 4 en une quantité suffisante pour lutter contre la croissance des mauvaises herbes.

6. Procédé pour préparer un éther diphénylique comme défini dans la revendication 1, qui comprend la réaction d'un composé de formule:

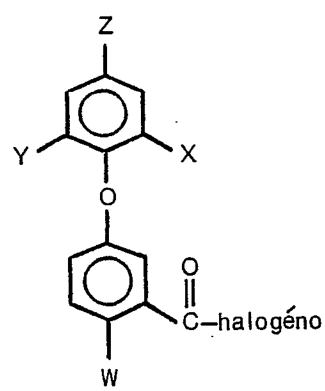

dans laquelle W, X, Y et Z sont comme défini dans la revendication 1, avec un composé de formule: $HONR^1(CHR^2)_nR^3$; dans laquelle $R^1$, $R^2$, $R^3$ et n sont comme défini dans la revendication 1, à une température dans la gamme d'environ 0°C à environ 50°C dans un solvant inerte et en présence d'un fixateur d'acide.